# EUROPEAN PATENT APPLICATION

(11) **EP 4 624 912 A1**
(43) Date of publication of application: **01.10.2025**
(21) Application number: 23894644.6
(22) Date of filing: 22.11.2023
(51) Int. Cl.: G01N 25/20, G01N 25/38, G01N 33/44

(54) **TEMPERATURE HISTORY ESTIMATION DEVICE, TEMPERATURE HISTORY ESTIMATION METHOD, AND TEMPERATURE HISTORY ESTIMATION PROGRAM**

(30) Priority: 25.11.2022 JP 2022188015
(71) Applicant: NOK Corporation, Minato-ku Tokyo 105-8585 (JP)
(72) Inventor: ANZAI Takahiro, Fujisawa-shi, Kanagawa 2510042 (JP); AOYAGI Yuichi, Fujisawa-shi, Kanagawa 2510042 (JP)
(74) Representative: Global IP Europe Patentanwaltskanzlei
(86) International application number: PCT/JP2023/042044
(87) International publication number: WO 2024/111640

(57) **Abstract**

A temperature history of a rubber composition is estimated by a simple method. A temperature history estimation device (1) estimates a temperature history of a measurement object that is a rubber composition. The temperature history estimation device (1) includes a measurement value reception unit (101) that receives a measurement value of a carbonization rate of a polymer contained in the measurement object, a storage unit (20) that stores data (202) showing a relationship between a carbonization rate of a polymer contained in a sample of the rubber composition, and a temperature, and a temperature history estimation unit (102) that estimates the temperature history of the measurement object based on the measurement value of the carbonization rate and the data (202).

## Description

### Technical Field

The present invention relates to a temperature history estimation device, a temperature history estimation method, and a temperature history estimation program.

### Background Art

Conventionally, for rubber compositions, the degradation degrees have been estimated by measuring structural changes by using FT-IR, and measuring rubber hardness changes (For example, see Non-Patent Literature 1).

### Document List

### Non-Patent Literature

Non-Patent Literature 1: Tetsuya Kawashima, Toshio Ogawa, "Failure of NBR Due to Thermal Degradation", "Journal of the Society of Rubber Industry, Japan, 2002, Vol. 75, No. 6", pp. 257-262, The Society of Rubber Industry, Japan

### Summary of Invention

### Technical Problem

However, because these changes are difficult to quantify, and errors are also large, it has been difficult to accurately estimate the temperature histories of the samples of rubber compositions.

The present invention is made in view of the aforementioned problem, and it is an object of the present invention to provide a technique of estimating a temperature history of a rubber composition by a simple method.

### Solution to Problem

In order to achieve the above-described object, a temperature history estimation device according to the present invention is a device that estimates a temperature history of a measurement object that is a rubber composition, and includes a measurement value reception unit that receives a measurement value of a carbonization rate of a polymer contained in the measurement object, a storage unit that stores data showing a relationship between a carbonization rate of a polymer contained in a sample of the rubber composition, and a temperature, and a temperature history estimation unit that estimates the temperature history of the measurement object based on the measurement value of the carbonization rate and the data.

In the temperature history estimation device according to one aspect of the present invention, the temperature history estimation unit associates the measurement value of the carbonization rate with the carbonation rate of the polymer contained in the sample in the data, and estimates the temperature history of the measurement object.

In the temperature history estimation device according to one aspect of the present invention, the measurement value of the carbonization rate is measured by using a thermogravimetry-differential thermal analysis device.

In order to achieve the above-described object, a temperature history estimation method according to the present invention is a method for estimating a temperature history of a measurement object that is a rubber composition, and executes a step of receiving a measurement value of a carbonization rate of a polymer contained in the measurement object, and a step of estimating the temperature history of the measurement object based on the measurement value of the carbonization rate and data showing a relationship between a carbonization rate of a polymer contained in a sample of the rubber composition and a temperature.

In order to achieve the above-described object, a temperature history estimation program according to the present invention is a program for a computer to execute a process of estimating a temperature history of a measurement object that is a rubber composition, the program causing the computer to execute a step of receiving a measurement value of a carbonization rate of a polymer contained in the measurement object, and a step of estimating the temperature history of the measurement object based on the measurement value of the carbonization rate, and data showing a relationship between a carbonization rate of a polymer contained in a sample of the rubber composition and a temperature.

In the temperature history estimation method, and the temperature history estimation program according to one aspect of the present invention, in the step of estimating the temperature history of the measurement object, the measurement value of the carbonization rate is associated with the carbonization rate of the polymer contained in the sample in the data, and the temperature history of the measurement object is estimated.

In the temperature history estimation method, and the temperature history estimation program according to one aspect of the present invention, the measurement value of the carbonization rate is measured by using a thermogravimetry-differential thermal analysis device.

### Effects of Invention

According to the temperature history estimation device, the temperature history estimation method, and the temperature history estimation program according to the present invention, it is possible to estimate the temperature history of a rubber composition by a simple method.

### Brief Description of Drawings

[Fig. 1] A functional block diagram of a temperature history estimation device according to an embodiment of the present invention.
[Fig. 2] A sectional perspective view of a gear damper having an example of a rubber composition for which estimation of a temperature history is performed by the temperature history estimation device according to the present embodiment.
[Fig. 3] A schematic view showing an example of a screen that receives input of a measurement value of a carbonization rate, which is displayed on a notification unit by a measurement value reception unit in the temperature history estimation device according to the present embodiment.
[Fig. 4] A graph showing a TG-DTA curve in the rubber composition for which estimation of the temperature history is performed by the temperature history estimation device according to the present embodiment.
[Fig. 5] A schematic diagram for explaining a principle of degradation of a rubber composition.
[Fig. 6] A graph showing a relationship between a carbonization rate of polymers and temperature history in the rubber composition for which estimation of the temperature history is performed by the temperature history estimation device according to the present embodiment.
[Fig. 7] A graph showing a relationship between a rubber hardness change and a temperature history, in the rubber composition.
[Fig. 8] A graph showing a relationship between FT-IR and the temperature history in the rubber composition.
[Fig. 9] A schematic view showing an example of a screen for displaying an estimation result of a temperature history that is calculated based on the measurement value, which is displayed on the notification unit by the temperature history estimation unit in the temperature history estimation device according to the present embodiment.
[Fig. 10] Flowchart for explaining a temperature history estimation method executed by the temperature history estimation device according to the present embodiment.

### Description of Embodiment

Hereinafter, an embodiment of the present invention will be described with reference to the drawings.

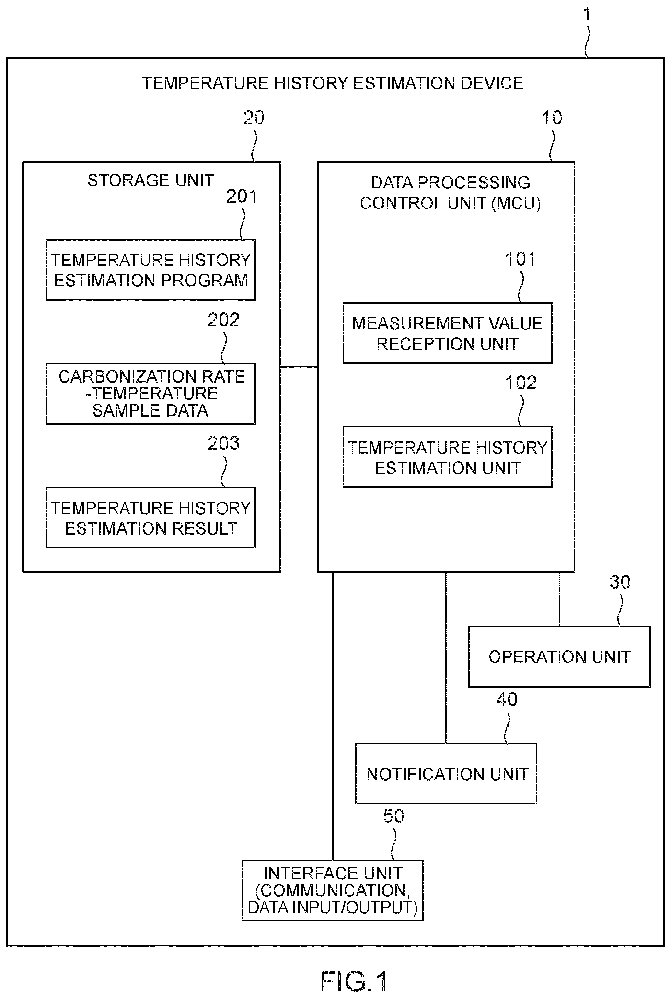

Fig. 1 is a functional block diagram of a temperature history estimation device 1 according to the embodiment of the present invention.

As shown in Figure 1, the temperature history estimation device 1 according to the embodiment of the present invention is, for example, an information processing device including a data processing control unit 10, and a storage unit 20, such as a PC (Personal Computer), a smartphone, and a tablet device.

The data processing control unit 10 is configured by an information processing apparatus capable of executing a computer program, such as an MCU (Micro Controller Unit) which includes a processor such as a CPU (Central Processing Unit) and a storage device. The storage device included in the data processing control unit 10 is implemented by, for example, a known storage device (storage medium) such as a ROM, a RAM, or a flash memory.

The data processing control unit 10 is a functional unit that integrally controls each functional unit in the temperature history estimation device 1. As described above, the data processing control unit 10 is configured by including, for example, the processor such as a CPU. The data processing control unit 10 controls each functional unit in the temperature history estimation device 1 by executing various arithmetic operations according to a program stored in the storage unit 20, for example.

The storage unit 20 is a nonvolatile storage device that stores various types of information described later, and is, for example, a magnetic storage device such as a hard disk drive, or an electrical storage device such as a solid state drive.

Various programs for causing the information processing device to realize functions as the temperature history estimation device 1 are stored in the storage unit 20. The various programs stored in the storage unit 20 include a temperature history estimation program 201 according to the present invention. Furthermore, in the storage unit 20, data (Hereinafter, referred to as "carbonization rate-temperature sample data 202".) is stored, which shows a relationship between a temperature and a carbonization rate of a polymer contained in a sample of a rubber composition having a composition corresponding to a measurement object, and is to be used in an arithmetic operation for estimating a temperature history of the measurement object. Furthermore. the storage unit 20 is a functional unit for storing various data such as a temperature history estimation result 203.

An operation unit 30 is an input interface for a user to operate the temperature history estimation device 1. Examples of the operation unit 30 include various buttons, a keyboard, a pointing device, a touch panel and the like. For example, by operating the operation unit 30 by the user, it is possible to set various conditions and the like for estimating the temperature history of the measurement object to the temperature history estimation device 1, and instruct the temperature history estimation device 1 to execute and stop a temperature history estimation process. Note that the operation unit 30 is not limited to the aforementioned buttons or touch panel, and it is sufficient as long as the operation unit 30 has a function of being capable of receiving an input for the user to operate the temperature history estimation device 1. The operation unit 30 may receive an operation by a command input by a voice, for example.

The notification unit 40 is a functional unit for notifying the user of information by outputting various types of information such as specification of the measurement object in the temperature history estimation device 1, confirmation of a name of the carbonization rate-temperature sample data 202 used in estimation process of the temperature history and data that is stored, estimation conditions, and an estimation result. The notification unit 40 is, for example, a display device including an LCD (Liquid Crystal Display), or an organic EL. Furthermore, the notification unit 40 may be a device that outputs audio data and notifies the user of the information, such as a speaker or a headphone. Note that the notification unit 40 may be a display device including a touch panel that realizes a part of the function as the operation unit 30.

An interface unit 50 is configured by various interfaces that enable input and output of the data between the temperature history estimation device 1 and an external device not illustrated. The interface unit 50 may be, for example, various communication interfaces such as a LAN, a USB, and an RS-232C. By the interface unit 50, the temperature history estimation device 1 enables input and output of data via the above-described external device. The interface unit 50 may include a communication circuit or the like that outputs various data used for estimating the temperature history to the outside by wire or wirelessly. By these various communication interfaces, the temperature history estimation device 1 can communicate with, for example, a wide area network (WAN: Wide Area Network) represented by the Internet or a local area network (LAN: Local Area Network).

The temperature history estimation device 1 includes the above hardware configuration, and the data processing control unit 10 executes the temperature history estimation program stored in the storage unit 20, whereby the hardware and software cooperate with each other to perform a process of estimating the temperature history of the measurement object that is a rubber composition. The temperature history estimation device 1 includes a measurement value reception unit 101, the aforementioned storage unit 20, and a temperature history estimation unit 102 as functional blocks implemented by the temperature history estimation program.

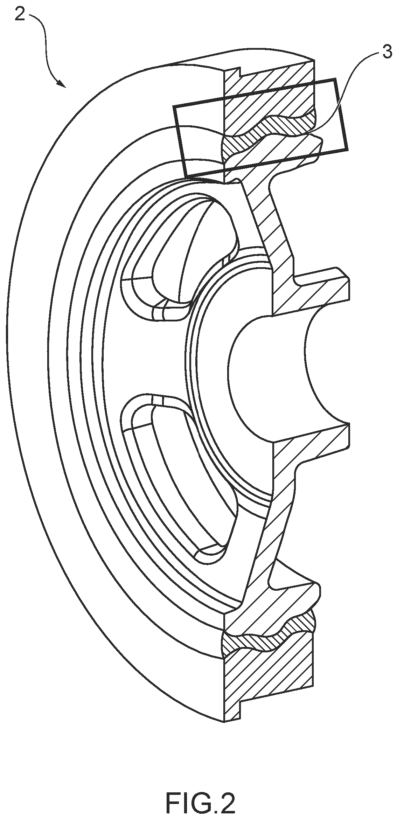

Fig. 2 is a sectional perspective view of a gear damper 2 having an example of the rubber composition for which estimation of the temperature history is performed by the temperature history estimation device 1 according to the present embodiment. In the present embodiment, the temperature history estimation device 1 estimates a value of a temperature of heat to which this rubber composition has been subjected, that is, the temperature history by measuring a carbonization rate of the polymer contained in the rubber composition such as a rubber ring 3 provided in the gear damper 2, for example, a hydrogenated nitrile rubber (HNBR).

Note that the rubber composition as the measurement object for which the estimation process of the temperature history is performed by the temperature history estimation device 1 can be used in various rubber products without being limited to those used for an anti-vibration rubber product such as the gear damper shown in Fig. 2 or a torsional damper.

The measurement value reception unit 101 receives the measurement value of the carbonization rate of the polymer contained in the measurement object (Hereinafter, referred to as a "measurement value".). The measurement value is measured by using a thermogravimetry-differential thermal analysis device (TG-DTA: Thermogravimetry-Differential Thermal Analysis) which is a device that simultaneously measures thermogravimetric analysis and differential thermal analysis. Specifically, from a weight reduction amount in a combustion region of the rubber (about 450 to 550° in the air), the amount of rubber structurally changed and carbonized due to the temperature history is measured.

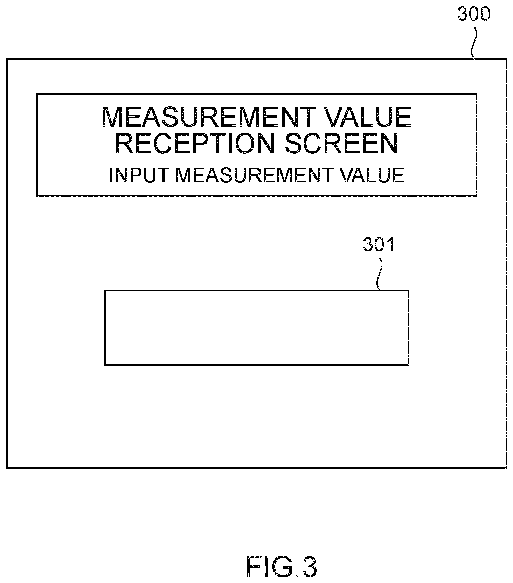

Fig. 3 is a schematic view showing an example of a measurement value reception screen 300 that receives input of the measurement value of the carbonization rate, which is displayed in the notification unit 40 by the measurement value reception unit 101 in the temperature history estimation device 1 according to the present embodiment. The measurement value reception screen 300 is displayed in a display device that forms the notification unit 40 (see Fig. 1), for example. The user can input the measurement value, for example, in a measurement value input unit 301 in the measurement value reception screen 300 via the operation unit 30 or the interface unit 50. The measurement value inputted in the measurement value input unit 301 is received by the measurement value reception unit 101. Note that an input method of the measurement value is not limited to the aforementioned example.

The temperature history estimation unit 102 estimates the temperature history of the measurement object based on the measurement value received by the measurement value reception unit 101, and the carbonization rate-temperature sample data 202 stored in the storage unit 20. Specifically, the temperature history estimation unit 102 estimates the temperature history of the measurement object by associating the measurement value of the carbonization rate received by the measurement value reception unit 101 with the carbonization rate of the polymer included in the sample in the carbonization rate-temperature sample data 202.

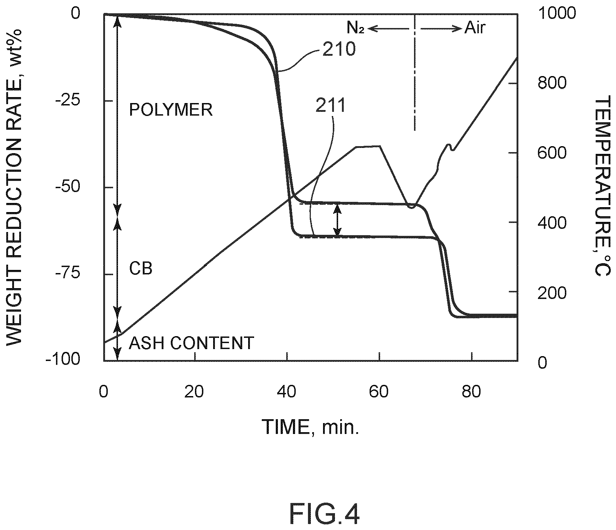

Fig. 4 is a graph showing a TG-DTA curve in the rubber composition for which estimation of the temperature history is performed by the temperature history estimation device 1 according to the present embodiment. In Fig. 4, a vertical axis on a left side represents a weight reduction rate (wt%), a vertical axis on a right side represents a temperature (°C), and a horizontal axis represents a time (minute), respectively. In Fig. 4, a TG-DTA curve 210 in a rubber composition of a new product (not placed in a high-temperature environment), and a TG-DTA curve 211 in a rubber composition subjected to heat at a predetermine temperature (for example, 150°C) are shown. In the present embodiment, the rubber composition used under the high-temperature environment is an object for which a temperature history is estimated, that is, the rubber composition to be the measurement object.

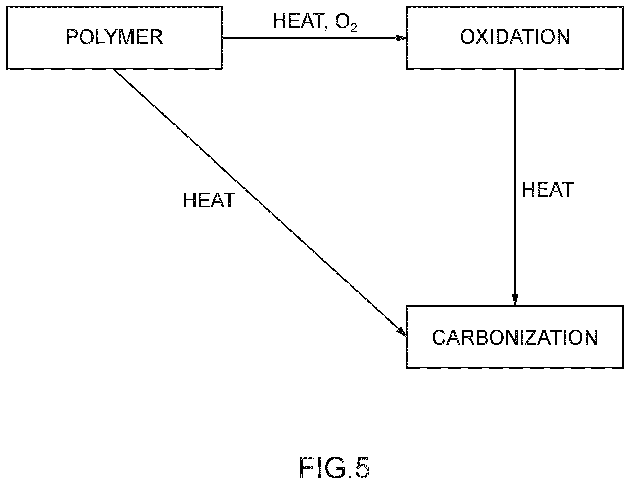

Fig. 5 is a schematic diagram for explaining a principle of degradation of the rubber composition. As can be seen from the TG-DTA curve 210 in the rubber composition of the new product shown in Fig. 4, and the TG-DTA curve 211 in the rubber composition subjected to the heat at a predetermined temperature, in the rubber composition, a weight percent of the carbide (carbon precursor) increases by the rubber composition being used under the high-temperature environment. As shown in Fig. 5, the polymer in the rubber composition such as HNBR is estimated to be cyclized in a high-temperature environment and altered to the carbide (carbon precursor). Therefore, in the temperature history estimation device 1, the temperature history of the measurement object is estimated based on the carbonization rate of the polymer contained in the measurement object of the rubber composition.

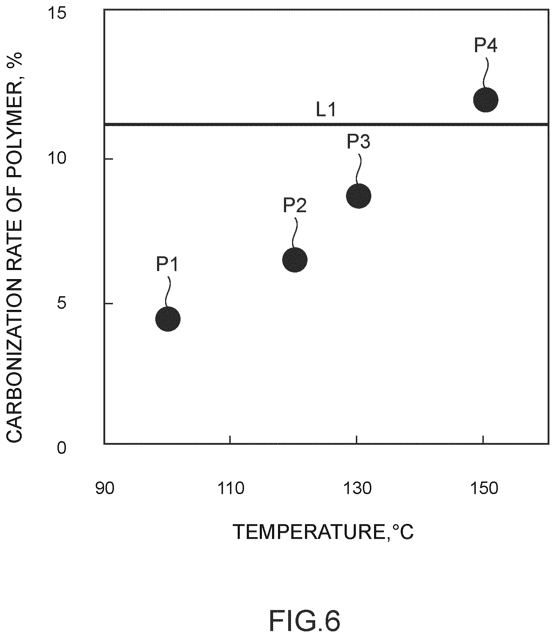

Fig. 6 is a graph showing a relationship between the carbonization rate of the polymer and the temperature history in the rubber composition for which estimation of the temperature history is performed by the temperature history estimation device 1 according to the present embodiment. In Fig. 6, a vertical axis represents a carbonization rate [%] of the polymer, and a horizontal axis represents the temperature history of the rubber composition. As shown in Fig. 6, it can be found that in the rubber composition for which estimation of the temperature history is performed by the temperature history estimation device 1, there is a relationship in which as the carbonization rate of the polymer rises, the temperature indicated by the temperature history to which the rubber composition containing the polymer has been subjected also rises. That is to say, it can be found that if the carbonization rate of the polymer can be measured in the rubber composition, the temperature history to which the rubber composition has been subjected can be estimated. In the graph showing the relationship between the carbonization rate of the polymer and the temperature history like this, a line L1 shows a predetermined temperature and the carbonization rate as a reference in determining degradation of the rubber composition. It can be found that among an arbitrary number of points plotted in the graph, for example, points P1 to P4 in Fig. 5, the point P4 above the line L1 is a temperature history exceeding the above-described predetermined temperature.

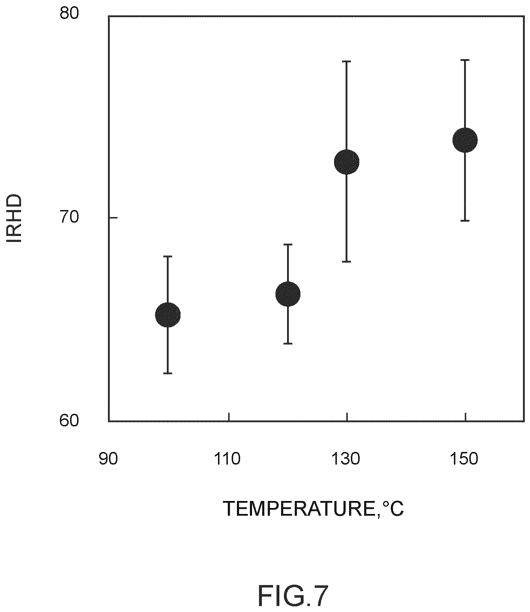

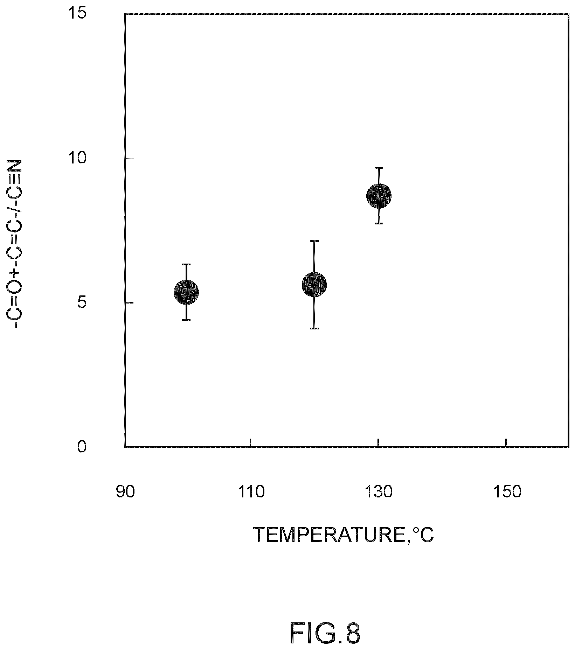

Fig. 7 is a graph showing a relationship between a rubber hardness change and the temperature history in the rubber composition. Furthermore, Fig. 8 is a graph showing a relationship between FT-IR and the temperature history, in the rubber composition.

In evaluation using the rubber hardness change shown in Fig. 7, and evaluation using FT-IR (Fourier Transform-Infrared Spectroscopy) shown in Fig. 8, there is a large variation between each value measured from the rubber composition and the temperature history to which the rubber composition is subjected. Therefore, it is difficult to estimate the temperature history of the rubber composition by using the rubber hardness change and the FT-IR. Furthermore, in the case of estimating the temperature history from the structural change by the FT-IR, the structural change at a high temperature cannot be captured from the measured value, and standardization is difficult.

Therefore, the temperature history estimation device 1 stores, in the storage unit 20, the combination of the value of the temperature history and the carbonization rate of the polymer in the temperature history, measured in advance from the sample of the rubber composition having an equivalent composition to the that of the measurement object, as the carbonization rate-temperature sample data 202. The carbonization rate-temperature sample data 202 is, for example, stored in the storage unit 20 for each type of the rubber composition. In the carbonization rate-temperature sample data 202, the temperature history associated with the carbonization rate can be stored in the storage unit 20 at an interval of any value (step) such as every 20°C of the temperature history or every 5% carbonization rate, for example.

The temperature history estimation device 1 determines the value of the temperature history corresponding to the measurement value of the carbonization rate received from the measurement value reception unit 101 from the carbonization rate-temperature sample data 202 stored in the storage unit 20. In this manner, the temperature history estimation device 1 can estimate the temperature history of the rubber composition by measuring the carbonization rate of the polymer of the rubber composition.

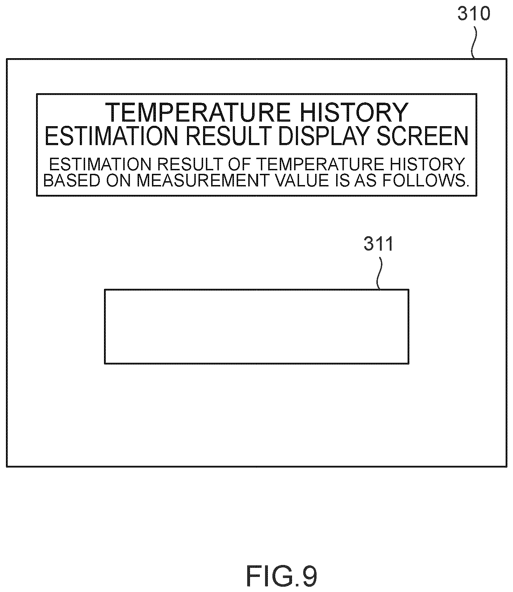

Fig. 9 is a schematic view showing an example of a temperature history estimation result display screen 310 that displays an estimation result of the temperature history calculated based on the measurement value, which is displayed on the notification unit 40 by the temperature history estimation unit 102 in the temperature history estimation device 1 according to the present embodiment. The temperature history estimation result display screen 310 is displayed on the display device that forms the notification unit 40, for example. The user can recognize the estimation result of the temperature history, for example, by confirming a numeric value displayed on an estimation result display unit 311 in the temperature history estimation result display screen 310. Note that a display method and an output method of the estimation result of the temperature history is not limited to the aforementioned example.

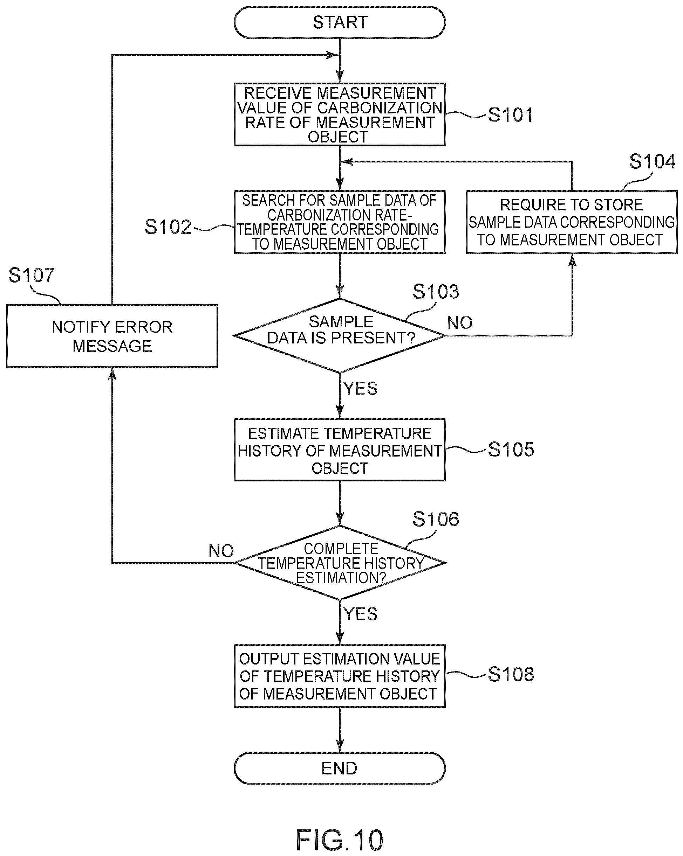

Fig. 10 is a flowchart for explaining a temperature history estimation method executed by the temperature history estimation device 1 according to the present embodiment.

First, in the temperature history estimation device 1, the measurement value reception unit 101 receives an input of the measurement value of the carbonization rate of the polymer of the measurement object from the operation unit 30 or the interface unit 50 (step S101).

The temperature history estimation unit 102 searches, in the storage unit 20, for the carbonization rate-temperature sample data 202 of the rubber composition corresponding to the measurement value of the measurement object received by the measurement value reception unit 101, that is, having the equivalent composition (step S102).

The temperature history estimation unit 102 determines whether or not the carbonization rate-temperature sample data 202 corresponding to the measurement value of the measurement object is present in the storage unit 20 (step S103). When the corresponding carbonization rate-temperature sample data 202 is not present in the storage unit 20 (S103: NO), the temperature history estimation unit 102 notifies the user to store the corresponding carbonization rate-temperature sample data 202 via the notification unit 40 (step S104). After S104, the temperature history estimation unit 102 returns to the process in S102.

When the corresponding carbonization rate-temperature sample data 202 is present in the storage unit 20 (S103: YES), the temperature history estimation unit 102 estimates the temperature history of the measurement object based on the measurement value of the measurement object, and the carbonization rate-temperature sample data 202 (step S105).

The temperature history estimation unit 102 determines whether or not the estimation process of the temperature history of the measurement object can be completed by associating the measurement value of the measurement object with the value of the carbonization rate in the carbonization rate-temperature sample data 202 (step S106). When the estimation process of the temperature history of the measurement object cannot be completed (S106: NO), the temperature history estimation unit 102 informs an error message indicating that the temperature history estimation process is not completed from the notification unit 40 (step S107) and returns to the process in S101.

When the estimation process of the temperature history of the measurement object can be completed (S106: YES), the temperature history estimation unit 102 outputs the estimation value of the temperature history of the measurement object from the notification unit 40 (step S108). After the process in S108, the temperature history estimation device 1 ends the process.

As described above, in the temperature history estimation device 1 according to the present embodiment, it is possible to execute the temperature history estimation method that estimates the temperature history of the rubber composition based on the measurement value of the carbonization rate of the polymer contained in the rubber composition of the measurement object by executing the temperature history estimation program.

Specifically, in the temperature history estimation method which the temperature history estimation device 1 according to the present embodiment executes, attention is paid to the fact that when rubber (NBR) degrades and the structure of the rubber changes, the degradation and the change of the structure of the rubber occur via a carbon precursor. In the temperature history estimation method, the amount of the precursor of carbon, that is, the carbonization rate of the polymer was measured by using TG-DTA, and the temperature history of the measurement object was estimated based on the relationship between the carbonization rate and the temperature history. Here, it is possible to use the temperature history of the rubber composition as an index of a degradation degree. TG-DTA has smaller error in the relationship between each value and the temperature history as compared to FT-IR and the rubber hardness change. Furthermore, TG-DTA can make it easier to quantify as compared to FT-IR and the rubber hardness change.

Consequently, according to the temperature history estimation method executed by the temperature history estimation device 1, it is possible to estimate the temperature history of the rubber composition by the simple method.

Although the embodiment of the present invention is described above, the present invention is not limited to the embodiment of the present invention described above, and includes all aspects included in the concept and the claims of the present invention. Furthermore, in order to exhibit at least a part of the aforementioned effect, the respective configurations may be selectively combined as appropriate. For example, the shape, material, disposition, size and the like of each component in the above embodiment can be changed as appropriate, according to the specific usage aspect of the present invention.

### List of Reference Signs

1: temperature history estimation device,
2: gear damper,
3: rubber ring,
10: data processing control unit,
20: storage unit,
30: operation unit,
40: notification unit,
50: interface unit,
101: measurement value reception unit,
102: temperature history estimation unit,
201: temperature history estimation program,
202: temperature sample data,
203: temperature history estimation result,
210, 122: TG-DTA curve,
300: measurement value reception screen,
301: measurement value input unit,
310: temperature history estimation result display screen,
311: estimation result display unit

## Claims

1. A temperature history estimation device that is a device that estimates a temperature history of a measurement object that is a rubber composition, comprising:
a measurement value reception unit that receives a measurement value of a carbonization rate of a polymer contained in the measurement object;
a storage unit that stores data showing a relationship between a carbonization rate of a polymer contained in a sample of the rubber composition, and a temperature;
and
a temperature history estimation unit that estimates the temperature history of the measurement object based on the measurement value of the carbonization rate and the data.

2. The temperature history estimation device according to claim 1, wherein the temperature history estimation unit associates the measurement value of the carbonization rate with the carbonation rate of the polymer contained in the sample in the data, and estimates the temperature history of the measurement object.

3. The temperature history estimation device according to claim 1 or 2, wherein the measurement value of the carbonization rate is measured by using a thermogravimetry-differential thermal analysis device.

4. A temperature history estimation method that is a method for estimating a temperature history of a measurement object that is a rubber composition, the method executing
a step of receiving a measurement value of a carbonization rate of a polymer contained in the measurement object; and
a step of estimating the temperature history of the measurement object based on the measurement value of the carbonization rate and data showing a relationship between a carbonization rate of a polymer contained in a sample of the rubber composition and a temperature.

5. The temperature history estimation method according to claim 4,
wherein in the step of estimating the temperature history of the measurement object, the measurement value of the carbonization rate is associated with the carbonization rate of the polymer contained in the sample in the data, and the temperature history of the measurement object is estimated.

6. The temperature history estimation method according to claim 4 or 5,
wherein the measurement value of the carbonization rate is measured by using a thermogravimetry-differential thermal analysis device.

7. A temperature history estimation program that is a program for a computer to execute a process of estimating a temperature history of a measurement object that is a rubber composition, the program causing the computer to execute
a step of receiving a measurement value of a carbonization rate of a polymer contained in the measurement object; and
a step of estimating the temperature history of the measurement object based on the measurement value of the carbonization rate, and data showing a relationship between a carbonization rate of a polymer contained in a sample of the rubber composition and a temperature.

8. The temperature history estimation program according to claim 7,
wherein in the step of estimating the temperature history of the measurement object, the measurement value of the carbonization rate is associated with the carbonization rate of the polymer contained in the sample in the data, and the temperature history of the measurement object is estimated.

9. The temperature history estimation program according to claim 7 or 8,
wherein the measurement value of the carbonization rate is measured by using a thermogravimetry-differential thermal analysis device.
